# EUROPÄISCHE PATENTSCHRIFT

(11) **EP 1 198 580 B1**
(45) Veröffentlichungstag und Bekanntmachung des Hinweises auf die Patenterteilung: **31.05.2006**
(21) Anmeldenummer: 00951400.1
(22) Anmeldetag: 18.07.2000
(51) Int. Cl.: C12N 15/864, C12N 15/35, C12N 15/62, C07K 14/015, C07K 19/00, C12N 5/10, C12Q 1/68, G01N 33/68, A61K 39/23, A61K 48/00

(54) **STRUKTURPROTEIN VON ADENO-ASSOZIIERTEM VIRUS MIT VERÄNDERTEN CHROMATOGRAPHISCHEN EIGENSCHAFTEN**
SCLEROPROTEIN OF AN ADENO-ASSOCIATED VIRUS WITH MODIFIED CHROMATOGRAPHIC PROPERTIES, THE PRODUCTION THEREOF AND USE OF THE SAME
PROTEINE STRUCTURALE DE VIRUS ADENO-ASSOCIE A PROPRIETES CHROMATOGRAPHIQUES MODIFIEES, SA PRODUCTION ET SON UTILISATION

(30) Priorität: 19.07.1999 DE 19933719
(43) Veröffentlichungstag der Anmeldung: 24.04.2002
(73) Patentinhaber: MediGene AG, 82152 Martinsried (DE)
(72) Erfinder: HALLEK, Michael, D-50935 Köln (DE); GIROD, Anne, D-82287 Jesenwang (DE); RIED, Martin, D-86697 Sinning (DE); STOLLA, Christof, CH-4057 Basel (CH); MOEBIUS, Ulrich, D-82131 Gauting-Unterbrunn (DE)
(74) Vertreter: Bösl, Raphael Konrad
(86) Internationale Anmeldenummer: PCT/EP2000/006861
(87) Internationale Veröffentlichungsnummer: WO 2001/005991

(56) Entgegenhaltungen:
- WO-A-96/00587
- WO-A-97/38723
- WO-A-99/67393
- YANG Q. ET AL.: "DEVELOPMENT OF NOVEL CELL SURFACE CD34-TARGETED RECOMBINANT ADENOASSOCIATED VIRUS VECTORS FOR GENE THERAPY" HUMAN GENE THERAPY, Bd. 9, Nr. 13, 1. September 1998 (1998-09-01), Seiten 1929-1937, XP000867311 ISSN: 1043-0342
- VALSESIA-WITTMANN S. ET AL.: "MODIFICATIONS IN THE BINDING DOMAIN OF AVIAN RETROVIRUS ENVELOPE PROTEIN TO REDIRECT THE HOST RANGE OF RETROVIRAL VECTORS" JOURNAL OF VIROLOGY, Bd. 68, Nr. 7, 1. Juli 1994 (1994-07-01), Seiten 4609-4619, XP000616602 ISSN: 0022-538X in der Anmeldung erwähnt
- WICKHAM T. J. ET AL.: "Increased in vitro and in vivo gene transfer by adenovirus vectors containing chimeric fiber proteins" JOURNAL OF VIROLOGY, Bd. 11, Nr. 71, 1. November 1997 (1997-11-01), Seiten 8221-8229, XP002078898 ISSN: 0022-538X
- GIROD A. ET AL.: "GENETIC CAPSID MODIFICATIONS ALLOW EFFICIENT RE-TARGETING OF ADENO-ASSOCIATED VIRUS TYPE 2" NATURE MEDICINE, Bd. 5, Nr. 9, September 1999 (1999-09), Seiten 1052-1056, XP002128040 ISSN: 1078-8956 in der Anmeldung erwähnt

## Beschreibung

Die vorliegende Erfindung betrifft ein Strukturprotein von Adeno-assoziiertem Virus, das mindestens eine Mutation enthält, die eine Veränderung der chromatographischen Eigenschaften bewirkt, seine Herstellung und Verwendung.

Das AAV-Virus gehört zur Familie der Parvoviren. Diese zeichnen sich durch ein ikosaedrisches, unbehülltes Kapsid mit einem Durchmesser von 18 bis 30 nm aus, welches eine lineare, einzelsträngige DNA von ca. 5 kb enthält. Für eine effiziente Vermehrung von AAV ist eine Coinfektion der Wirtszelle mit Helferviren, beispielsweise mit Adenoviren, Herpesviren oder Vacciniaviren erforderlich. In Abwesenheit eines Helfervirus geht AAV in einen Latenzzustand über, wobei das Virusgenom in der Lage ist, stabil in das Wirtszellgenom zu integrieren. Die Eigenschaft von AAV, in das Wirtsgenom zu integrieren, macht es als Transduktionsvektor für Säugetierzellen besonders interessant. Für die Vektorfunktionen genügen im allgemeinen die beiden ca. 145 bp langen invertierten terminalen Wiederholungssequenzen (ITR: "Inverted Terminal Repeats"). Sie tragen die "cis" notwendigen Signale für Replikation, Verpackung und Integration in das Wirtszellgenom. Zur Verpackung in rekombinante Vektorpartikel wird ein Helferplasmid, welches die Gene für nicht-strukturelle Proteine (Rep-Proteine) und für strukturelle Proteine (Cap-Proteine) trägt, in verpackungsgeeignete Zellen, z.B. HeLa- oder 293-Zellen, transfiziert, die hierauf beispielsweise mit Adenovirus infiziert werden. Nach einigen Tagen erhält man ein Lysat, welches rekombinante AAV-Partikel enthält. Geeignete Helferplasmide sind z.B. bei Chiorini et al., (1995) Hum. Gene Ther. 6, 1531-1541 oder Girod et al. (1999), Nat. Med. beschrieben.

Das AAV-Kapsid besteht aus drei verschiedenen Proteinen: VP1, VP2 und VP3, deren relative Anteile 5% VP1, 5% VP2 und 90% VP3 sind. Die AAV-Kapsidgene sind am rechten Ende des AAV-Genoms lokalisiert und werden durch überlappende Sequenzen desselben offenen Leserahmens (ORF) unter Verwendung verschiedener Startkodons sowie zweier unterschiedlich gespleißter Transkripte kodiert. Das VP1-Gen enthält die ganze VP2-Gensequenz, welche wiederum die ganze VP3-Gensequenz mit einem spezifischen N-terminalen Bereich enthält. Die Tatsache, daß die überlappenden Leserahmen für alle drei AAV-Kapsid-Proteine kodieren, ist für die obligatorische Expression aller Kapsid-Proteine, wenn auch zu unterschiedlichen Anteilen, verantwortlich.

Die Molekularmassen der Kapsid-Proteine sind 87 kD für VP1, 73 kD für VP2 und 62 kD für VP3. Die Sequenzen der Kapsidgene sind in Srivastava, A. et al. (1983), J. Virol., 45, 555-564; Muzyczka, N. (1992), Curr. Top. Micro. Immunol., 158, 97-129, Ruffing, N. et al. (1992), J. Virol., 66, 6922-6930 oder Rutledge, E. A. et al. (1998) J. Virol. 72, 309-319 beispielsweise beschrieben. Die physikalische und genetische Karte des AAV-Genoms ist beispielsweise bei Kotin, R. M. (1994), Human Gene Therapy, 5, 793-801 beschrieben.

Zudem sind verschiedene AAV-Serotypen bekannt, von denen der menschliche AAV-Serotyp 2 (AAV2) am besten untersucht ist. In diesen Analysen zeigte sich, daß AAV Viren als virale Vektoren vorteilhafte Eigenschaften für die somatische Gentherapie besitzen. Die wesentlichen Vorteile sind die fehlende Pathogenität für den Menschen, die stabile Integration viraler DNA in das zelluläre Genom, die Fähigkeit, nicht teilende Zellen zu infizieren, die Stabilität des Virions, was die Aufreinigung zu hohen Titern (10¹³ bis 10¹⁴ Partikel pro ml) ermöglicht, die geringe Antigenität sowie das Fehlen viraler Gene und Genprodukte im rekombinanten AAV-Vektor, was unter Sicherheitsaspekten für die Verwendung in der Gentherapie vorteilhaft ist. Die Klonierung von Genen in den AAV-Vektor erfolgt mittlerweile nach den dem Fachmann allgemein bekannten Methoden, wie sie z.B. in WO 95/ 23 867, bei Chiorini, J. A. et al. (1995), Human Gene Therapy, 6, 1531-1541 oder bei Kotin, R. M. (1994), supra, beschrieben sind.

Für die Verwendung von AAV als viralen Transduktionsvektor werden im allgemeinen hohe Titer an rekombinanten AAV-Partikeln benötigt. Durch die naturgegebene, verhältnismäßig geringe Produktion von Partikeln ist ein Weg, hohe Titer zu erzielen, eine effiziente Anreicherung der Partikel. Des weiteren müssen die Partikel insbesondere für in vivo Anwendungen möglichst frei sein von Verunreinigungen, die aus zellulären Bestandteilen, DNA, Proteine, Helferviren und Mediumbestandteilen bestehen können. Somit ist es notwendig, eine verbesserte Aufreinigung für AAV-Partikel zur Verfügung zu haben.

Ein grundlegende Möglichkeit zur Aufreinigung bietet die Chromatographie. Bei diesem physikalischen Trennverfahren erfolgt die Stofftrennung durch Verteilung zwischen einer stationären und einer mobilen Phase. Anhand der physikalischen Vorgänge läßt sich die Chromatographie in zwei Gruppen einteilen, die Adsorptionschromatographie mit einem Feststoff als stationärer Phase und die Verteilungschromatographie bei zwei nicht miteinander mischbaren Phasen, wobei meist Mischformen vorkommen. Das Trennverhalten eines Stoffes in der Chromatographie hängt dabei von seinen chromatographischen Eigenschaften ab, insbesondere seiner Größe, seiner Ladung, seinem Adsorptionsverhalten, so seiner spezifischen Affinität, seiner Hydrophobizität, etc. Damit bieten die chromatographischen Eigenschaften einen zentralen Ansatzpunkt, um über eine Veränderung eine Verbesserung der Aufreinigung und damit beispielsweise eine Anreicherung oder höhere Reinheit zu erzielen, wobei bereits eine reine Veränderung, beispielsweise gegenüber dem Wildtyp, eine Trennung und damit bessere Aufreinigung erlauben kann.

Aufreinigungsverfahren für AAV, insbesondere mittels Chromatographie, sind beispielsweise in WO 97/08298 beschrieben, eine Mutation der Strukturproteine von AAV aber nicht. Des weiteren wird in WO 96/00587 auf AAV-Kapsid-Fusionsproteine hingewiesen, die mit der Kapsidbildung nicht interferieren und heterologe Epitope klinisch relevanter Antigene enthalten sollen, wodurch aber lediglich eine Immunantwort induziert werden soll. Die Veröffentlichung enthält außerdem nur einen allgemeinen Hinweis auf die Fusionsproteine ohne nähere Angaben zur Ausführbarkeit, insbesondere zu geeigneten Insertionsstellen. Eine Änderung chromatographischer Eigenschaften insbesondere zur Verbesserung der Aufreinigung, z.B. durch Änderung der Affinitäten, wird aber nicht beschrieben.

Aufgabe der vorliegenden Anmeldung war es daher, die Reinigungseigenschaften des AAV-Virus, insbesondere eines Strukturproteins, gegenüber dem Wildtyp zu verändern.

Überraschenderweise wurde nun gefunden, daß Struktur- bzw. Kapsid-Proteine von AAV so modifiziert werden können, daß dadurch eine Änderung der chromatographischen Eigenschaften bewirkt wird.

Ein Gegenstand der vorliegenden Erfindung betrifft daher die Verwendung eines Strukturproteins von Adeno-assoziiertem Virus (AAV) für die Aufreinigung von AAV und/oder AAV-Partikeln, wobei das Strukturprotein mindestens eine Mutation in Form einer Insertion enthält, die eine Änderung der chromatographischen Eigenschaften des Virus bewirkt und die nach mindestens einer Aminosäure in der Sequenz ausgewählt aus YKQIS SQSGA, YLTLN NGSQA, YYLSR TNTPS, EEKFF PQSGV, NPVAT EQYGS, LQRGN RQAAT, NVDFT VDTNG lokalisiert ist. Dabei ist bevorzugt, daß die Änderung der chromatographischen Eigenschaften eine Verbesserung der Aufreinigung ermöglicht, insbesondere eine Anreicherung des Virus, bevorzugt der Virus-Partikel, zu höheren Titern, eine Aufreinigung zu größerer Reinheit und/oder eine effizientere Aufreinigung. Durch die veränderten chromatographischen Eigenschaften kann beispielsweise ein spezifischerer bzw. effizienterer Reinigungsschritt für Virus-Partikel im Rahmen einer Aufreinigung erzielt werden, der zu höheren Partikeltitern, zu reinerenPartikeln oder zu einer effizienteren Aufreinigung führt. Der Titer rekombinanter Partikel kann z.B. dadurch bestimmt werden, daß eine Partikel-enthaltende Lösung in einer Verdünnungsreihe auf eine Membran gegeben und diese Membran mit markierter AAV-DNA hybridisiert wird. Durch den Nachweis der hybridisierten DNA kann auf die Partikelkonzentration je nach Durchführung des Versuchs qualitativ oder quantitativ rückgeschlossen werden. Die Reinheit der Partikel kann durch das Verhältnis des Strukturproteins bzw. der Partikelproteine zu partikelfremden Proteinen bestimmt werden. Eine effizientere Aufreinigung liegt im Sinne der vorliegenden Erfindung dann vor, wenn die Aufreinigung beispielsweise aus weniger Schritten besteht, schneller verläuft oder insbesondere für eine großtechnische Anwendung billiger in der Durchführung ist.

Im Sinne dieser Erfindung liegt eine Änderung der chromatographischen Eigenschaften des Virus verbunden mit einer Verbesserung der Aufreinigung beispielsweise bereits dann vor, wenn die Mutation lediglich eine Verschiebung des Elutionsverhaltens auf einer Chromatographiesäule bewirkt, also beispielsweise zu niedrigeren oder höheren Salzkonzentrationen hin. Allgemeines Problem bei chromatographischen Reinigungen ist, daß die gewünschten Produktfraktionen (z.B. Virus- partikel) und Fraktionen von Verunreinigungen (andere Viren, Wildtyp-Viren, Reste von Zellysaten, DNA, Proteine, insbesondere Serumproteine) bei gleichem Salzgehalt in der gleichen Fraktion eluiert werden. Durch eine erfindungsgemäße gezielte Mutation und die damit verbundene Verschiebung des Elutionsverhaltens eluiert die Fraktion mit den gewünschten, mutierten Viruspartikeln dann aber nicht mehr mit der verunreinigten Fraktion sondern in einer anderen Elutionsfraktion (z.B. - je nach Ladung - bei höheren oder niedrigeren Salzkonzentrationen). Unter Umständen, aber nicht immer, bieten dabei Verschiebungen zu hohen Salzkonzentrationen, beispielsweise durch Insertion überwiegend positiv oder negativ geladener Aminosäuren oder His-TAG in das Kapsidprotein, besondere Vorteile, da Verunreinigungen meist kleine Bestandteile sind und bei üblichen Reinigungsverfahren meist bei geringeren Salzkonzentrationen eluieren. Die Kapsidmutanten binden dann beispielsweise besser an das Säulenmaterial und eluieren später, also bei höheren Salzkonzentrationen. Ein weiterer erwünschter Effekt aufgrund des Einbaus von geladenen Aminosäuren ist die Möglichkeit, Ionenaustauscher bei einem höheren Salzgehalt zu beladen, wodurch die Menge des benötigten Säulenmaterials reduziert werden kann, was die Handhabung erleichtert sowie in einem industriellen Herstellungsverfahren Kosten spart.

Es ist besonders bevorzugt, daß die Mutation im erfindungsgemäßen Strukturprotein keine wesentliche Verringerung der Infektiosität des Virus, insbesondere aber auch eine Erhöhung der Infektiosität, bewirkt. Unter Infektiosität versteht man im Sinne dieser Erfindung die Fähigkeit, Zellen zu transduzieren.

Eine weitere Ausgestaltung dieser Erfindung ist, daß das modifizierte Strukturprotein gegenüber dem Wildtyp-AAV eine erhöhte Hitzestabilität aufweist. So wäre bei erhöhter Hitzestabilität eine bessere Hitzeinaktivierung von unerwünschten anderen Mikroorganismen oder Viren möglich, als dies für Wildtyp-AAV der Fall ist. Derartige Strukturporteine ließen sich einfach dadurch ausfindig machen, in dem eine Vielzahl von Mutanten hinsichtlich ihrer Hitzestabilität getestet werden.

Des weiteren ist das modifizierte Strukturprotein vorzugsweise weiterhin zur Partikelbildung, d.h. zur Ausbildung eines ikosaedrischen Kapsids, befähigt, insbesondere in Form eines AAV-Kapsids, da Partikel bzw. Kapside als Träger von ausgewählten Verbindungen, z.B. rAAV-Transduktionsvektoren, besonders geeignet sind. Die Bildung von Partikeln läßt sich beispielsweise durch Elektronenmikroskopie nachweisen. Ein anderer Nachweis ist das Sedimentationsverhalten während einer Cäsiumchlorid-Dichtegradientenzentrifugation mit anschließendem, optionalen Nachweis von in den Partikeln enthaltener viraler DNA.

Im allgemeinen kann (können) die Mutation(en) im VP1-, VP2- und/oder VP3-Strukturprotein liegen, wobei das VP1- und/oder das VP3-Strukturprotein bevorzugt sind. Des weiteren kann das Strukturprotein von allen AAV-Serotypen abgeleitet sein, insbesondere von humanen Serotypen, vorzugsweise von AAV1, AAV2, AAV3, AAV4, AAV5 und/oder AAV6, vor allem von AAV2, AAV3 und/oder AAV6.

In einer bevorzugten Ausführungform werden Aminosäuren einer funktionellen Sequenz insertiert, die für die Affinitätschromatographie geeignet sind.

Unter Affinitätschromatographie versteht man eine Methode der Chromatographie, die auf der Fähigkeit bestimmter zusammengehöriger Partner wie Antigen-Antikörper, Enzym-Substrat etc. beruht, sich gegenseitig zu erkennen und miteinander in Wechselwirkung zu treten. Dabei wird meist einer der zusammengehörigen Partner an ein chromatographisches Sorbens als Träger fixiert, an den dann die spezifisch passende Komponente bindet. Anschließend wird mit verändertem pH, anderer Ionenstärke oder z.B. Analogen der passenden Komponente eluiert. Umfaßt ist damit auch die kovalente Chromatographie z.B. über Bildung von Disulfid-Brücken und die hydrophobe Chromatographie, bei der hydrophobe Wechselwirkungen ausgenutzt werden.

Insbesondere kann die insertierte Aminosäure aus folgender Gruppe ausgewählt sein: einem Liganden eines Rezeptors oder dem Rezeptor eines Liganden, einem Antikörper oder Teil eines Antikörpers, insbesondere einem Antikörper-Epitop, einem Antigen oder Antigen-Epitop, einem Hormon, einem Hormorezeptor, einem Enzym, einem Enzymsubstrat, einem Lektin und/oder einer zucker-tragenden Aminosäure.

Vorzugsweise können dies sein:
- ein Histidin-reiches Peptid (His-TAG), das eine Aufreinigung über ein MetallChelat-Affinitätsmedium ermöglicht;
- ein Peptid mit mehreren Ladungen, das das Bindungs- bzw. Elutionsverhalten während einer Ionenaustauschchromatographie verändert und so einen derartigen Reinigungsschritt spezifischer oder effizienter gestaltet;
- die Glutathion-S-Transferase (GST-Tag), das eine Aufreinigung über ein Glutathion-Affinitätsmedium ermöglicht;
- ein F_{c}-Teil eines Antikörpers, der eine Aufreinigung über ein Protein A- oder Protein G-Affinitätsmedium ermöglicht;
- eine Immunglobulin-bindende Domäne, beispielsweise Protein A oder Protein G bzw. Teile davon, das eine Aufreinigung über ein Affinitätsmedium mit einem Antikörper oder einem F_{c}-Teil eines Antikörpers ermöglicht;
- ein bestimmtes Antikörper-Epitop, das eine Aufreinigung über ein Medium mit gekoppelten Antikörpern ermöglicht, die für das Epitop spezifisch sind;
- ein Lecitin, das eine Aufreinigung über ein Glycoprotein-Medium ermöglicht;
- eine Nukleinsäure-Bindestelle, die eine Aufreinigung über Nukleinsäure-Medien ermöglicht;
- eine Heparin-Bindestelle, die eine Aufreinigung über ein Heparin-Medium ermöglicht, wobei eine intrinsische Heparin-Bindungsstelle in Wildtyp-AAV bereits vorhanden ist, so daß eine zusätzliche Bindungsstelle die Bindung lediglich verstärken würde;
- Streptavidin, das eine Aufreinigung über Biotin oder biotinylierte Proteine ermöglicht;
- ein bestimmter Ligand, der eine Aufreinigung über ein Medium mit dem entsprechenden Rezeptor ermöglicht oder
- ein bestimmter Rezeptor, der eine Aufreinigung über ein Medium mit dem entsprechenden Liganden ermöglicht.

Ebenfalls bevorzugt sind ein Integrin, ein Cytokin oder eine Rezeptor-Bindungsdomäne von einem Cytokin, Integrin oder Wachstumsfaktor, an einem Zelloberflächenrezeptor bindende einzelkettige Antikörper, ein Antikörper gegen Zelloberflächenstrukturen, ein Epitop und/oder eine antikörperbindende Struktur.

In einer bevorzugten Ausführung wird ein Peptid mit beispielsweise 5 bis 30 Aminosäuren, vorzugsweise 8 bis 20 Aminosäuren und insbesondere 10 bis 18 Aminosäuren insertiert. Das Peptid hat beispielsweise die Sequenz QAGTFALRGDNPQG oder eine Sequenz, die zu dieser stark homolog ist. Bei diesem besonders bevorzugten Liganden handelt es sich um das P1-Peptid, das ein 14 Aminosäuren langes Peptid aus der Core-Sequenz einer Alpha-Kette der Laminin-Familie darstellt. Diese Sequenz ist beispielsweise ausreichend, um einen Integrin-Rezeptor zu erkennen, der u.a. die Endozytose viraler Partikel, z.B. von Adenovirus, vermittelt. Das P1-Peptid bindet unabhängig von seiner Konformation (linear oder zirkulär) an den Integrin-Rezeptor. Gemäß der vorliegenden Erfindung wird die kodierende DNA-Sequenz des P1-Peptids in das Gen kodierend für ein Strukturprotein von AAV, welches beispielsweise auf einem Helferplasmid liegt, eingebaut. Nach der Verpackung mit dem mutanten Helferplasmid erhält man rekombinantes AAV mit P1 im Kapsid (rAAV-P1). Für die Insertion dieses Peptids konnte gezeigt werden, daß diese AAV-Partikel im Vergleich zu unmodifizierten AAV-Partikeln bereits bei niedrigerer Leitfähigkeit von einem Kationenaustauscher eluieren und so, je nach Bedingungen, eine verbesserte Trennung (z.B. vom Wildtyp) und Aufreinigung erlauben.

Besonders bevorzugt ist ein erfindungsgemäßes Strukturprotein, das mindestens eine weitere Mutation enthält. Darunter ist zu verstehen, daß das Strukturprotein neben einer Mutation, die eine Veränderung der chromatographischen Eigenschaften des Virus bewirkt, auch eine weitere Mutation enthält, die nicht zwangsläufig auch eine Veränderung der chromatographischen Eigenschaften des Virus bewirkt. Besonders bevorzugt ist hier eine weitere Mutation, die eine Änderung, vorzugsweise Erhöhung, der Infektiosität des Virus bewirkt.

Ein weiterer bevorzugter Gegenstand der vorliegenden Erfindung ist ein Strukturprotein von AAV, bei dem die weitere(n) Mutation(en) eine Verringerung der Antigenität bewirkt (bewirken).

Unter Antigenität versteht man im Sinne dieser Erfindung die Auslösung sowohl einer Antikörperbildung als auch -bindung aufgrund des Immunsystems. Der Begriff umfaßt auch die Immunogenität, also die Auslösung einer Immunantwort. Unter der Verringerung der Antigenität versteht man daher die Verringerung der Antikörperbildung und -bindung sowohl durch Verringerung der antigenen Epitope, als auch durch Verringerung der antigenen Wirkung bestimmter Epitope oder durch Veränderung und Entfernung bestimmter im Wildtyp vorhandener Epitope. Die veränderte Antigenität kann sich dabei sowohl auf die humorale wie auch die zelluläre Immunantwort beziehen.

In einer weiteren bevorzugten Ausführungsform stellt/stellen die weiteren Mutation/en eine oder mehrere Deletionen und/oder eine oder mehrere Insertionen im Strukturprotein oder Kombinationen der genannten Modifikationen dar. Dabei ist die Insertion vorzugsweise die Insertion eines Zellmembranrezeptor-Liganden, eines Rep-Proteins oder -Peptids, beispielsweise in Form einer Rep-Domäne, eines immunsuppressiven Proteins oder Peptids und/oder eines Proteins oder Peptids mit einem Signal zur Doppelstrangsynthese eines Transgens bzw. Fremdgens. Bevorzugt ist hierbei beispielsweise das P1-Peptid (QAGTFALRGDNPQG) (s.o).

Beispiele von Insertionen bei der weiteren Mutation sind u.a. Integrine, Cytokine oder Rezeptor-Bindungsdomänen von Cytokinen, Integrinen oder Wachstumsfaktoren, wie z.B. GM-CSF, IL-2, IL-12, CD40L, TNF, NGF, PDGF oder EGF, an Zelloberflächenrezeptoren bindende einzelkettige Antikörper, sog. "single chain" Antikörper (scFv), beispielsweise an die Oberflächenrezeptoren CD40, CD40L, B7, CD28 oder CD34 bindende einzelkettige Antikörper, oder Epitope bzw. Rezeptorbindungsstellen, die beispielsweise ihrerseits von bestimmten Antikörpern, beispielsweise Anti-CD40L-monoklonale Antikörper, bzw. von chemischen Substanzen oder Hormonen, z.B. Katecholamine, erkannt werden.

In einer bevorzugten Ausführungsform der weiteren Mutation werden antikörperbindende Strukturen, wie z.B. Protein A, Protein G oder anti-Fc-Antikörper, bzw. Teile hiervon, insertiert. An diese werden wiederum spezifische Antikörper gegen bestimmte Zelloberflächenstrukturen (beispielsweise gegen das CD40 bei lymphatischen Zellen oder gegen das CD34 bei hämatopoietische Zellen) angekoppelt.

Vorzugsweise ist/sind die Mutation/en an der Virusoberfläche lokalisiert. Zur Bestimmung der oberflächen-lokalisierten Bereiche der Strukturproteine wurde gemäß der vorliegenden Erfindung überraschenderweise gefunden, daß CPV- und AAV2-Sequenzen und -Strukturen vergleichbar sind. Man kann daher vorzugsweise auf bekannte Kristallstrukturen von Parvoviren wie von Parvovirus B19 oder von CPV (Canine-Parvovirus) zurückgreifen und mit Hilfe von Homologievergleichen Proteindomänen identifizieren, die auf der Virusoberfläche lokalisiert sind. Gemäß der vorliegenden Erfindung hat daher beispielsweise ein computerunterstützter Vergleich zwischen CPV und AAV2 bzw. Parvovirus B 19 und AAV2 überraschenderweise reproduzierbar zur Identifikation von Schleifen (Loops) in VP3 geführt, deren Sequenz variiert, d.h. die eine geringe Homologie besitzen und die voraussichtlich auf der Virusoberfläche lokalisiert sind. Da die Antigene für die humorale Immunantwort für Antikörper zugänglich und somit auf der Virusoberfläche sein müssen, stellen diese Schleifen bevorzugte Kandidaten für Mutationen dar. So wurde die bekannte Kristallstruktur des CPV VP2-Kapsid-Proteins (z.B. Luo M.(1988), J. Mol. Biol., 200, 209-211; Wu und Rossmann (1993), J.Mol.Biol., 233, 231-244) aufgrund der hohen Ähnlichkeit zum AAV2 VP3 in der sekundären Struktur des Proteins als Muster genommen, um die Regionen herauszufinden, die auf der viralen Kapsidoberfläche exponiert sind und die aufgrund der lokalen Aminosäuresequenz flexibel genug sind, beispielsweise die Insertion einer Peptidsequenz zu überstehen. Dabei wurde sorgfältig darauf geachtet, daß keine sekundären Strukturelemente des AAV2-Kapsidproteins ausgewählt wurden, die das Kapsid destabilisieren würden.

In einer bevorzugten Ausführungsform sind die Mutation(en) am N-Terminus des Strukturproteins lokalisiert, da gefunden wurde, daß beispielsweise bei den Parvoviren CPV und B 19 der N-Terminus an der Zelloberfläche liegt.

Eine weitere Möglichkeit zur Bestimmung der Oberflächen-lokalisierten Bereiche der Strukturproteine ist ein Vergleich der für die Kapside kodierenden Nukleinsäuresequenzen von unterschiedlichen AAV-Serotypen. Hierzu können beispielsweise bekannte DNA-Sequenzen unterschiedlicher AAV-Serotypen, wie AAV1, AAV2, AAV3, AAV4, AAV5 oder AAV6, für Strukturanalysen möglicher Kapsidmorphologien von beispielsweise AAV2 herangezogen werden, wobei ab initio mögliche Tertiärstrukturen berechnet und Sequenzbereiche aufgrund allgemein bekannter Aminosäure-Eigenschaften den inneren oder äußeren Kapsidbereichen zugeordnet werden können. So konnten beispielsweise gemäß der vorliegenden Erfindung mögliche Insertionsstellen im VP3-Bereich des AAV2-Kapsids ermittelt werden, die die Insertion beispielsweise von Peptiden und deren Expression auf der Virusoberfläche ermöglichten (siehe unten).

In einer weiteren bevorzugten Ausführungsform liegen ein oder mehrere Insertionen im VP3-Stmkturprotein (Rutledge, E. A. et al. (1998) supra) vor und/oder nach mindestens einer Aminosäure in der Sequenz ausgewählt aus YKQIS SQSGA, YLTLN NGSQA, YYLSR TNTPS, EEKFF PQSGV, NPVAT, EQYGS, LQRGN RQAAT, NVDFT VDTNG, da diese Stellen an den exponierten Stellen eines Loops liegen, wobei das Risiko gering ist, die VP3-Struktur zu ändern.

Die Insertion(en) wird/werden nach allgemein bekannten Methoden durch Insertion in dem für das Strukturprotein codierenden Gen durchgeführt. Die Insertionen lassen sich nach allgemein bekannten Methoden beispielsweise mittels Hydrolyse durch Restriktionsendonukleasen der entsprechenden Strukturprotein-Gene und anschließender Ligasereaktion einfügen. Die anschließende Expression des mutierten Gens führt zum erfindungsgemäßen Strukturprotein.

Ein Gegenstand der vorliegenden Erfindung ist auch die erfindungsgemäße Verwendung des Struktur proteins in Form eines AAV-Partikels, insbesondere in Form eines AAV-Kapsids, da Partikel bzw. Kapside als Träger von ausgewählten Verbindungen, z.B. rAAV-Transduktionsvektoren, besonders geeignet sind.

Die vorliegende Beschreibung betrifft auch eine Nukleinsäure, vorzugsweise eine RNA oder DNA, insbesondere eine doppelsträngige DNA, kodierend für ein erfindungsgemäßes Strukturprotein, und eine Zelle, vorzugsweise eine Säugetierzelle beispielsweise eine COS-Zelle, HeLa-Zelle oder 293-Zelle, enthaltend die betreffende Nukleinsäure. Derartige Zellen eignen sich beispielsweise zur Herstellung der rekombinanten AAV-Partikel.

Die vorliegende Beschreibung betrifft daher auch ein Verfahren zur Herstellung des betreffenden Strukturproteins, insbesondere zur Herstellung des betreffenden Strukturproteins in Form eines AAV-Partikels, wobei eine geeignete Zelle, enthaltend eine Nukleinsäure, kodierend für das entsprechende Strukturprotein kultiviert und ggf das exprimierte Strukturprotein isoliert wird. Beispielsweise läßt sich das entsprechende Struktuprotein chromatographisch reinigen und isolieren.

Die vorliegende Beschreibung bezieht sich auch auf ein Arzneimittel, enthaltend das betreffende Strukturprotein, eine entsprechende Nukleinsäure und/oder eine entsprechende Zelle und ggf. geeignete Hilfs- und Zusatzstoffe, wie z.B. eine physiologische Kochsalzlösung, Stabilisatoren, Proteinase-, DNAse-Inhibitoren, etc.

Ein weiterer Gegenstand der vorliegenden Erfindung bezieht sich auf die Verwendung des betreffender Strukturproteins, für die Aufreinigung von AAV und AAV-Partikeln, für die Änderung des Tropismus von AAV, für die Änderung der Antigenität von AAV, für die Transformation einer Zelle, insbesondere einer Zelle, die vorher einer AAV-Infektion wenig zugänglich war, wie beispielsweise einer hämatopoetischen Zelle, für das genomische Targeting, für die Diagnostik, für Wirksamkeitsuntersuchungen und/oder - in Form von geeigneten rAAV-Vektoren - für die Gentherapie. Unter Gentherapie versteht man eine Therapieform, bei der durch die Einführung von Nukleinsäuren in Zellen ein Effektorgen, meist ein Protein, exprimiert wird. Man unterscheidet prinzipiell *In-vitro-* und *In-vivo-*Verfahren. In *In-vitro*-Verfahren werden Zellen aus dem Organismus entfernt und *ex-vivo* mit Vektoren transduziert, um anschließend wieder in denselben oder in einen anderen Organismus eingebracht zu werden. Bei der *In-vivo*-Gentherapie werden Vektoren, beispielsweise zur Bekämpfung von Tumoren, systemisch (z.B. über die Blutbahn) oder direkt in den Tumor, das Gewebe oder die Organe appliziert.

Ein wesentlicher Vorteil der vorliegenden Erfindung ist, daß durch die erfindungsgemäße Mutagenese von Strukturproteinen von AAV über die Veränderung der chromatographischen Eigenschaften neue Möglichkeiten für spezifischere Aufreinigungsverfahren im wesentlichen ohne Verlust der Verpackungseffilzienz rekombinanter AAV-Vektoren in das Kapsid des Virus geschaffen werden können. Damit sind die Voraussetzungen geschaffen, AAV oder AAV-Partikel zu höheren Titern und/oder größerer Reinheit aufzureinigen und die Reinigung effizienter und kostengünstiger zu gestalten. Dies wiederum ermöglicht die großtechnische Anwendung von rekombinanten AAV für Zelltransformationen und Gentherapien im kommerziellen Maßstab.

Die folgenden Figuren und Beispiele sollen die Erfindung näher erläutern, ohne sie zu beschränken.

### Beschreibung der Figuren

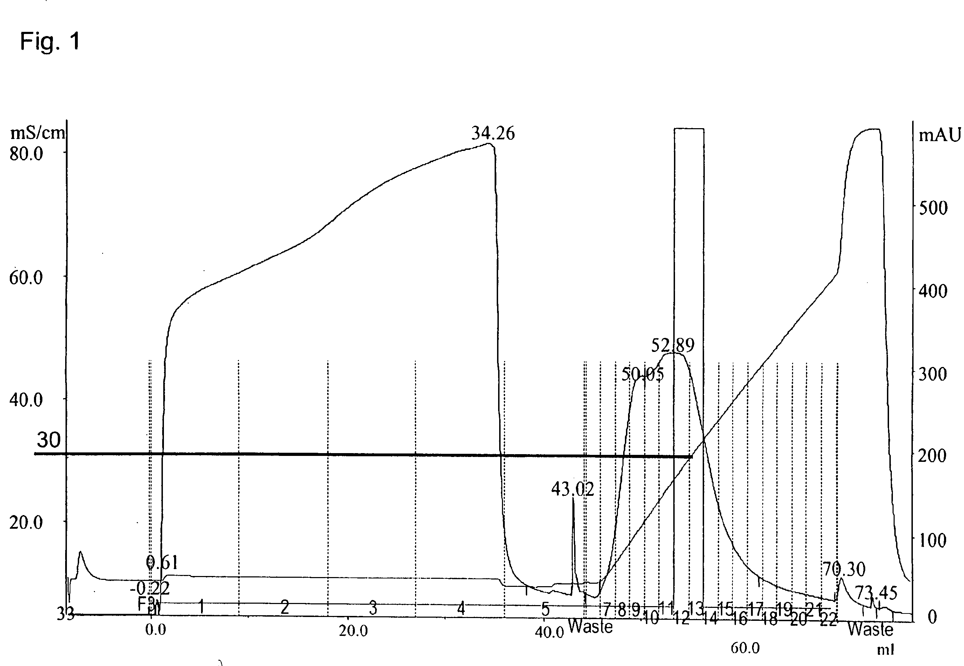

Figur 1 zeigt das Chromatogramm für eine Wildtyp-AAV-Probe in einem Lauf über eine POROS 50HS Kationenaustauschsäule. Aufgetragen ist das Durchflußvolumen gegen die Leitfähigkeit (linke y-Achse) und gegen die Absorption bei 280 nm (rechte y-Achse). Die AAV-Partikel eluierten in den Fraktionen 12 und 13, was im Mittel einer Leitfähigkeit von 30 mS/cm (300 mM NaCl) entspricht (siehe dicker horizontaler Strich).

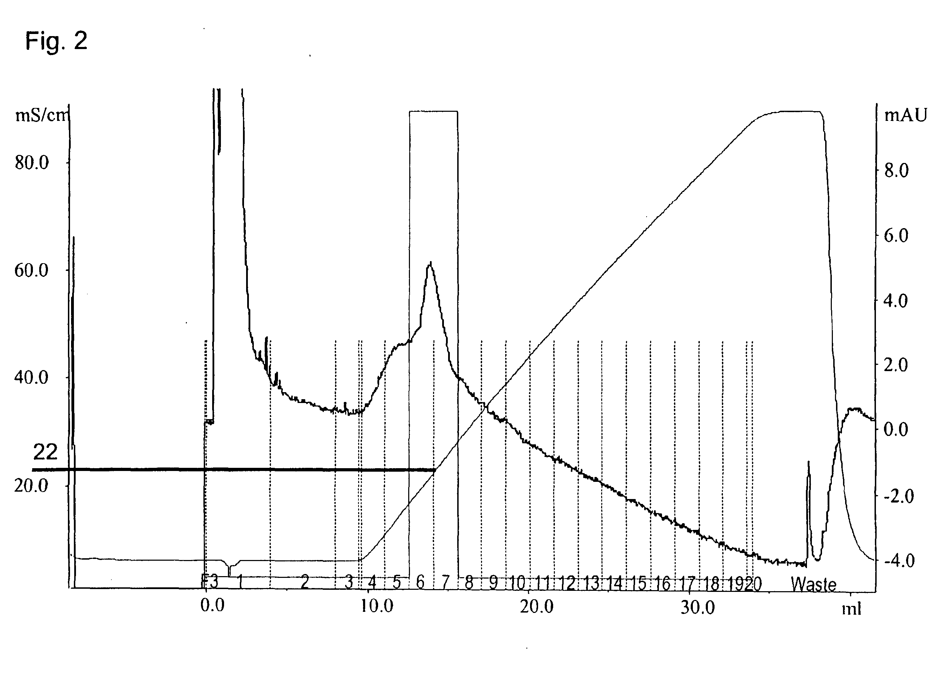

Figur 2 zeigt das Chromatogramm für eine AAV-Probe bestehend aus mutierten AAV-Partikeln (Insertion des Peptids QAGTFALRGDNPQG nach Aminosäure 587; 1-587 gemäß Beispiel 3) in einem Lauf über eine POROS 50HS Kationenaustauschsäule. Aufgetragen ist das Durchflußvolumen gegen die Leitfähigkeit (linke y-Achse) und gegen die Absorption bei 280 nm (rechte y-Achse). Die modifizierten AAV-Partikel eluierten in den Fraktionen 6 und 7, was im Mittel einer Leitfähigkeit von 22 mS/cm (220 mM NaCl) entspricht (siehe dicker horizontaler Strich).

### Beispiele

### Beispiel 1:

Mittels PCR-unterstützter Mutagenese und Schneiden mit den Restriktionsenzymen Xhol, BsrBI bzw. HindIII wurden folgende Mutationen hergestellt:

### Mutationen im VP1

a) Deletion zwischen den XhoI-XhoI-Schnittstellen des VP-1 (ΔXho; 62 Aminosäuren, AS) (Hermonat et al. (1984) Journal of Virology 51, 329 - 339),
b) Deletion zwischen BsrBI und HindII-Schnittstellen des VP-1, die innerhalb von der obigen Deletion a) liegt und 29 AS umfaßt (ΔBH);
c) Deletion zwischen BsrBI und HindII, sowie Insertion eines Liganden (P 1-Peptid) (ΔBH+L); und
d) Reine Insertion des Liganden (P1-Peptid) an der BsrBI-Schnittstelle (B+L).

### Mutationen im VP3

a) ins261; YKQIS SQSGA
b) ins381; YLTLN NGSQA
c) ins447; YYLSR TNTPS
d) ins534; EEKFF PQSGV
e) ins573; NPVAT EQYGS
f) ins587; LQRGN RQAAT
g) ins713; NVDFT VDTNG

(Nomenklatur nach Zahl der Aminosäuren (AS) gezählt nach den AS ab Beginn des N-Terminus im VP-1 von AAV2, umrahmt von jeweils 5 N-terminal davon gelegenen und 5 C-terminal davon gelegenen Aminosäuren; die AS, nach der die Insertion eingebracht wurde, ist in Fettschrift dargestellt).

Es ist auch möglich, daß in die fünf unmittelbar benachbarten AS, die neben der fett markierten AS liegen, ebenfalls eine Insertion eingebracht wird, da diese ebenfalls innerhalb eines Loops im AAV2-Kapsid liegen.

### Beispiel 2:

### Charakterisierung der Kapsidmutanten

Nach Durchführung der Mutationen im AAV2-Genom und Verpackung der mutierten Viren mit LacZ-Reportergen wurden die physikalischen Vektor-Titer durch Dot-Blot und Kapsid-Titer mit A20-Antikörper-ELISA bestimmt und erste Infektionstests auf HeLa-Zellen durchgeführt. Dadurch konnte bestimmt werden, ob die Mutationen die Struktur der VP-Proteine oder die Interaktion zwischen verschiedenen VP-Proteinen so stören, daß eine Verpackung unterbleibt bzw. gestört wird (Tabelle 1).

**Tabelle 1: Verpackungseffizienz der hergestellten Virusmutanten**

| Virusstock | genomische Virustiter | Kapsid-Titer (ELISA mit A20-MAb) |
|---|---|---|
| Wildtyp-Kapsid | 1.10¹² | 1-10¹¹ |

| VP1-Mutanten | | |
|---|---|---|
| Δxho | 6.10¹² | 5.10¹⁰ |
| ΔBH | 8.10¹¹ | 4.10⁹ |
| ΔBH+L | 1.10¹³ | 5.10¹⁰ |
| B+L | 3.10¹² | 5.10⁹ |

| VP3-Mutanten | | |
|---|---|---|
| ins261 1 | 1.10¹⁰ | < 10⁸ |
| ins381 | 3.10¹⁰ | < 10⁸ |
| ins447 | 1.10¹² | 4.10¹⁰ |
| ins534 | 1.10¹⁰ | < 10⁸ |
| ins573 | 3-10¹⁰ | < 10⁸ |
| ins587 | 1.10¹² | 2·10¹⁰ |
| ins713 | 5.10¹⁰ | < 10⁸ |

Gezeigt sind die genomischen Virustiter (Dot-Blot) und Kapsid-Titer (A20-Kapsid-ELISA). Die Konzentrationen sind in Partikel/ml angegeben.

Für alle 4 VP1-Mutanten konnte gezeigt werden, daß Mutationen die Verpackungseffizienz nicht beeinflussen und alle mutierten Viren mit ähnlichen guten Titern wie Vektoren mit unmutiertem Kapsid verpackt werden können (10¹¹ bis 10¹³ genomische Partikel/ml). Auch die AAV-Vektoren mit Mutationen im VP3-Bereich konnten erfolgreich verpackt werden (10¹⁰-10¹² genomische Partikel/ml).

### Beispiel 3

### P1-Mutation in VP3

Zunächst wurde von einem Plasmid pUC-AV2, das durch Subklonierung des 4.8-kb BgIII-Fragments des pAV2 (ATCC 37261, ref. 53) in die BamHI Schnittstelle des pUC19 (New England BioLabs Inc.) hergestellt wurde, ausgegangen. Mittels dem Fachmann bekannter PCR-unterstützter Mutagenese wurden an definierten Stellen des Plasmids Mutationen vorgenommen. Dabei wurde eine für P1, ein 14-AS-Peptid, mit der AS-Sequenz QAGTFALRGDNPQG, das das RGD-Bindungsmotiv eines Lamininfragments (Aumailly et al. (1990) FEBS Lett. 262, 82-86) enthält, codierende Sequenz nach den Nukleotiden 2985, 3543 und 3963 eingefügt. Dies entspricht einer Insertion nach den Aminosäuren 261, 447 und 587 des AAV2-Kapsidproteins (Nomenklatur nach Zahl der Aminosäuren (AS) gezählt nach den AS ab Beginn des N-Terminus im VP-1 von AAV2). In der anschließenden PCR werden jeweils 2 mutationsspezifische Primer und als Matrize ein Plasmid, pCap verwendet, das nur das cap-Gen enthält und dadurch gebildet wird, daß das 2.2 kb EcoRI-BspMI-Fragment aus pUC-Av2 herausgeschnitten und in die EcoRI-Schnittstelle des pUC 19 eingefügt wird. Anschließend werden die PCR Produkte in Bakterien amplifiziert, sequenziert und das 1.4-kb EcoNI-XcmI-Fragment, das P1 enthält in pUC-AV2, in dem die korrespondierende Wildtypcap-Sequenz herausgeschnitten wurde, subkloniert. Dementsprechend enthielten die nach den AS-Insertionsstellen pI-261, pI-381, pI-447 und pI-587 genannten Plasmide (Mutanten) das komplette AAV2-Genom. Die entsprechenden mutierten Proteine werden mit I-261, I-381, I-447 und I-587 bezeichnet.

### Beispiel 4

### Herstellung von AAV2-Partikeln

HeLa-Zellen (eine humane Cervix-Epithel-Zellinie) wurden mit den Plasmiden gemäß Beispiel 1 transfiziert, dann ca. 20h inkubiert und anschließend mit Adenovirus Typ 5 infiziert. 72 h nach der Infektion wurden die Zellen aufgeschlossen und die AAV2-Partikel über einen CsCI-Gradienten gereinigt.

### Beispiel 5

### Charakterisierung der Kapsidmutanten gemäß Beispiel 3

In diesen Versuchen sollte festgestellt werden, ob die Kapsidmutanten das virale Genom verpacken und vollständige Kapside bilden können. AAV2-Partikel der Mutanten gemäß Beispiel 4 wurden darauf überprüft, ob und wenn ja wieviele Partikel das Virus-Genom tragen und wieviel DNA in den Kapsid-Mutanten verpackt war. Dazu wurden die gemäß Beispiel 4 gereinigten Viruspartikel (Mutanten und Wildtyp) mit DNAse behandelt, geblottet und mit einer Rep-Sonde hybridisiert.

Der sich daraus ergebende Titer zeigte keine quantitative oder qualitative Differenz im Vergleich zum Wildtyp (s. Tabelle 2). Die Viren behielten die Fähigkeit, das Genom zu verpacken.

Durch Elektronenmikroskopanalyse konnte weiter bestätigt werden, daß auch das Kapsid ausgebildet wird.

Daher wurden die Mutationen nicht in Bereichen vorgenommen, die für die korrekte Faltung, die Kapsid-Zusammensetzung oder die Verpackung des Genoms von Bedeutung sind. Die erfindungsgemäßen AAV-Partikel sind in ihrer Funktion ungestört.

Um ablesen zu können, ob die mutierten Kapside vollständig gebildet werden und keine veränderte Antigenität zeigen, wurden in einem weiteren Experiment A20 monoklonale Antikörper (A20-MAb) in einem ELISA eingesetzt. A20-MAb reagieren spezifisch mit komplett zusammengesetztem AAV2-Kapsid des Wildtyps (Wistuba et al., (1997), J. Virol. 71, 1341-1352). Auch hier sind die Ergebnisse in Tabelle 2 dargestellt. Dabei zeigt sich, daß durch die Insertion in den Mutanten I-447 und 1-587 die Kapsid-Bildung nicht gestört wird, während bei I-261 der A20 monoklonale Antikörper nicht mehr bindet, was aber, da sich die Kapside nach Untersuchung mit dem Elektronomikroskop trotzdem bilden, auf eine Veränderung der Antigenität zurückzuführen ist.

**Tabelle 2 Verpackungseffizienz und Antigenität der hergestellten Virusmutanten gemäß Beispiel 3**

| Virusstock | genomische Virustiter | ELISA mit A20-MAb |
|---|---|---|
| Wildtyp-Kapsid | 8.10¹³ | 6.10¹² |

| Mutanten | | |
|---|---|---|
| I-261 | 1.10¹² | n.m. |
| 1-381 | 1.10¹² | n.m. |
| 1-447 | 1-10¹³ | 8-10¹¹ |
| I-587 | 4-10¹³ | 3. 10¹² |

Gezeigt sind die genomischen Virustiter (Dot-Blot) und der Titer mit A20-Kapsid-ELISA. Die Konzentrationen sind in Partikel/ml angegeben. "n.m." heißt "nicht meßbar".

### Beispiel 6:

### Verändertes Elutionsverhalten der Kapsidmutanten

Rekombinante Wildtyp-AAV (in 20 mM Hepes pH 6.8, 100 mM NaCl, 2 mM MgCl₂) wurden auf eine 0,8 ml POROS 20HS Kationenaustauschersäule (Perkin-Elmer, Weiterstadt) geladen. Es wurde mit Hilfe eines Äkta-Systems (Pharmacia) ein Gradient von 30 Säulenvolumina von 100 bis 700 mM NaCl in 20 mM Hepes 6,8 angelegt. Gemäß Western Blot Analyse eluierte AAV in den Fraktionen 12 und 13, was einer Elution von Wildtyp-AAV bei 30 mS/cm (=ca. 300 mM NaCl) entspricht (siehe Fig. 1).

Eine Kapsidmutante (1-587 gem. Beispiel 3) von AAV (das P1-Peptid QAGTFALRGDNPQG ist inseriert nach Aminosäure 587; in PBS, pH 6,8) wurde auf dieselbe 0,8 ml POROS 20HS Kationenaustauschersäule (siehe oben) aufgetragen. Es wurde in einem Äkta-System mit einem Gradienten von 30 Säulenvolumina 50-1000 mM NaCl in 20 mM Hepes pH 6,8 eluiert. Die AAV-Mutante befand sich gemäß Western Blot Analyse in den Fraktionen 6 und 7. Dies entspricht einer Elution von ca. 22 ms/cm (= ca. 220 mM NaCl) (siehe Fig. 2).

Dies zeigt, daß durch die Insertion des QAGTFALRGDNPQG-Peptids sich das Elutionsverhalten der AAV-Partikel ändert, so daß bei gleichem pH Wert die mutierten Partikel bei einer geringeren Salzkonzentration eluieren als die Wildtyp-Partikel. Damit wird die Virusfraktion zu anderen, unter Umständen weniger verunreinigten bzw. sonst geeigneteren Fraktionen hin verschoben. Daher ist es möglich, durch Insertionen, Deletionen oder sonstige Veränderungen der Kapsidproteine die chromatographischen Eigenschaften der AAV-Partikel zu ändern. Insbesondere können in einer Variante der gezeigten Insertion durch Einfügen von Aminosäuren mit überwiegend positiver Ladung, beispielsweise an den in den Beispielen gezeigten Insertionsstellen, erfindungsgemäße Kapsid-Mutanten konstruiert werden, die im Vergleich zum Wildtyp (der in einem breiten, weniger verunreinigten Peak eluiert) bei höheren Salzkonzentrationen eluieren. Die Affinität der Mutante zum Säulenmaterial ist dadurch verstärkt, so daß die Elution erst bei hohen Salzkonzentrationen erfolgt, also in Bereichen, die üblicherweise weniger durch kleinere Fremdproteine verunreinigt sind.

### Beispiel 7:

### Infektionstests mit Mutanten gemäß Beispiel 3

Um den Tropismus der Kapsidmutanten I-261, I-381, I-447 und I-587 zu testen, wurden Zellinien, Co-115 und B16F10, mit den mutierten Viren infiziert. Co-115-Zellen wurden zum Testen des Wildtyprezeptor-Tropismus der Virionen verwendet, da diese mit Wildtyp AAV2 transduziert werden können und das P1-Peptid nicht binden. Die B16F10-Zellinie wurde aus den in Beispiel 9 bereits genannten Gründen verwendet. Drei Tage nach der Infektion wurden die Zellen durch Immunofluoreszenzmessung mit Hilfe eines anti-Rep-Antikörpers darauf untersucht, ob das virale Rep-Protein exprimiert wird (Wistuba et al. (1997) J. Virol. 71, 1341-1352; Wistuba et al. (1995) J. Virol. 69, 5311-5319). Zellen wurden auf Objektträgern zu 70 % Konfluenz gezüchtet und mit verschiedenen Konzentrationen erfindungsgemäßer viraler Präparationen in serumfreiem Medium zusammen mit Adenovirus 5 inkubiert. Die Titer der viralen Präparationen wurden drei Tage später durch in-situ-Detektion der Rep-Proteinsynthese in einem Immunofluoreszenzassay (Rep-Titer) bestimmt. Dabei wurde die Immunofluoreszenzanfärbung mit AAV2-infizierten Zellen nach einer Methode von Wistuba et al. (Wistuba et al. (1997) J. Virol. 71, 1341-1352; Wistuba et al. (1995) J. Virol. 69, 5311-5319) durchgeführt. Die Objektträger wurden einmal mit PBS gewaschen, in Methanol (5 min, 4°C) fixiert und anschließend mit Aceton (5 min, 4°C) behandelt. Die Zellen wurden dann für eine Stunde bei Raumtemperatur mit dem monoklonalen Antikörper 76-3, der mit Rep-Proteinen von AAV2 reagiert, inkubiert. Anschließend wurde gewaschen und für eine Stunde mit einem Rhodamin-konjugierten Anti-Maus-sekundären Antikörper bei einer Verdünnung von 1:50 in PBS mit 1% BSA inkubiert. Die Titer wurden aus der letzten limitierenden Verdünnung der viralen Stammlösung errechnet, die zu fluoreszenzpositiven Zellen geführt hatten.

Rep-positive CO115-Zellen konnten nach Infektion mit Wildtyp AAV2 und mit Mutanten I-261, I-447 und I-587 detektiert werden. Bei Co115-Zellen war die Infektiosität von I-261, I-587 und 1-447 um zwei bis drei Größenordnungen kleiner als die des Wildtyps (Tabelle 3). Die Transfektion von B16F10-Zellen war mit I-447 genauso ineffektiv wie die mit Wildtypvirus (Tabelle 3). In klarem Gegensatz dazu sind nach Infektion mit I-587 Rep-positive B16F10-Zellen feststellbar, wobei der Titer des 1-587-Virus auf 1 x 10⁶ Rep-EFU/ml bestimmt wurde (Tabelle 3).

Um zu untersuchen, ob die Transfektion von B16F10-Zellen durch die Mutante 1-587 spezifisch durch die Interaktion zwischen der P1-Sequenz auf der Oberfläche des mutierten Kapsids und dem Integrinrezeptor auf der Oberfläche der B16F10-Zellen vermittelt wurde, wurden die Zellen entweder mit dem konkurrierenden RGDS oder dem inaktiven RGES-Peptid bei Konzentrationen von 200 µmol vor der Infektion mit dem Virus inkubiert. Die Zugabe von RGDS-Peptid neutralisierte die Infektiosität von 1-587 auf B16F10-Zellen (Tabelle 3), während das Kontrollpeptid RGES keinen Effekt hatte.

**Tabelle 3: Virustiter auf der Zelloberfläche**

| Virusstock | Titer auf CO115-Zellen | Titer auf B16F10-Zellen | |
|---|---|---|---|
| | | - RGDS | + RGDS |
| Wildtyp-Kapsid | 2.10⁹ | <1 | nb |

| Mutanten | | | |
|---|---|---|---|
| I-261 | 7.10⁶ | nb | nb |
| I-381 | n.m. | nb | nb |
| I-447 | 1·10⁶ | < 1 | nb |
| I-587 | 1·10⁷ | 1·10⁶ | <1 |
| rAAV/LacZ | 5·10⁷ | <1 | nb |
| rAAV(I-587)/LacZ | 6·10⁵ | 5·10⁴ | <1 |

Gezeigt sind die Titer auf den wildtypanfälligen CO115-Zellen und den wildtypresistenten B16F10-Zellen. Die Titer sind für I-447 und I-587 wie den Wildtyp in Rep-EFU/ml und für rAAV/LacZ und rAAV(I-587)/LacZ in LacZ-EFU/ml ausgedrückt. Dabei bedeutet EFU expressiorisbildende Einheiten (Expressing Forming Unit) und nb heißt "nicht bestimmt". "n.m." heißt "nicht meßbar".

### Beispiel 8:

### Infektionsassay der Mutanten gemäß Beispiel 3 mit Galaktosidase

In einem weiteren an Beispiel 6 angelehnten Versuch wurden rAAV-Vektoren mit einem LacZ-Reportergen hergestellt, die entweder den Wildtyp (rAAV-Virion) oder I-587 (rAAV(I-587)-Virion) enthielten. Die Viralpräparationen wurden rAAV/LacZ und rAAV(I-587)/LacZ genannt und zur Infektion von B16F10- bzw. CO115-Zellen (Kontrollen) verwendet.

Infizierte Zellen wurden drei Tage nach der Infektion durch X-Gal-Anfärbung auf β-Galaktosidase-Expression getestet. Dabei wurde der X-Gal-in-situ-Test zur zytochemischen Anfärbung (LacZ-Titer) verwendet. Nach diesem wurden die Zellen, um die Expression von β-Galaktosidase zu testen, einmal in PBS gewaschen und dann mit 1,5 % Glutaraldehyd fixiert. Anschließend wurden die Zellen mit X-Gal (5-bromo-4-chloro-3-indolyl-β-D-Galaktopyranosid) behandelt, wie bereits von Chiorini et al. (1995) Hum. Gen. Ther. 6, 1531-1541, beschrieben. Die Titer wurden aus der letzten limitierenden Verdünnung der viralen Stammlösung errechnet, die zu β-Galaktosidase-produzierenden Zellen geführt hatte.

Bei den Kontrollen an CO115-Zellen waren beide Virionen infektiös, allerdings rAAV (I-587)/LacZ um 2 Größenordnungen weniger effektiv. Bei Typ B16F10 wurden - wie erwartet - nach der Infektion mit rAAV/LacZ keine β-Galaktosidase-positiven Zellen gefunden. Nach der Infektion mit rAAV(I-587)/LacZ hingegen fanden sich überraschenderweise deutlich viele β-Galaktosidase-positive Zellen. Der Titer von rAAV-(I-587)/LacZ wurde mit 5 x 10⁴ LacZ-EFU pro ml bestimmt. Die Infektiosität von rAAV-Vektoren gegenüber B16F10-Zellen wurde durch die erfindungsgemäße Mutation um mehr als vier Größenordnungen verbessert (Tabelle 3).

### SEQUENZPROTOKOLL

<110> MediGene Aktiengesellschaft
<120> Strukturprotein von Adeno-assoziiertem Virus mit veränderten chromatographischen Eigenschaften, seine Herstellung und Verwendung
<140> 199 33 719.5-41
   <141> 17.07.1999
<160> 18
<170> Microsoft Word 97
<210> 1
   <211> 14
   <212> PRT
   <213> Mus musculus
<400> 1
<210> 2
   <211> 10
   <212> PRT
   <213> Adeno-assoziierter Virus
<400> 2
<210> 3
   <211> 10
   <212> PRT
   <213> Adeno-assoziierter Virus
<400> 3
<210> 4
   <211> 10
   <212> PRT
   <213> Adeno-assoziierter Virus
<400> 4
<210> 5
   <211> 10
   <212> PRT
   <213> Adeno-assoziierter Virus
<400> 5
<210> 6
   <211> 5
   <212> PRT
   <213> Adeno-assoziierter Virus
<400> 6
   Asn Pro Val Ala Thr
   5
<210> 7
   <211> 5
   <212> PRT
   <213> Adeno-assoziierter Virus
<400> 7
   Glu Gln Tyr Gly Ser
   5
<210>8
   <211>10
   <212> PRT
   <213> Adeno-assoziierter Virus
<400> 8
<210> 9
   <211> 10
   <212> PRT
   <213> Adeno-assoziierter Virus
<400> 9
<210> 10
   <211> 10
   <212> PRT
   <213> künstliche Sequenz
<220>
   <223> mutated VP3 of adeno-associated virus
<400> 10
<210> 11
   <211> 10
   <212> PRT
   <213> künstliche Sequenz
<220>
   <223> mutated VP3 of adeno-associated virus
<400> 11
<210> 12
   <211> 10
   <212> PRT
   <213> künstliche Sequenz
<220>
   <223> mutated VP3 of adeno-associated virus
<400> 12
<210> 13
   <211> 10
   <212> PRT
   <213> künstliche Sequenz
<220>
   <223> mutated VP3 of adeno-associated virus
<400> 13
<210> 14
   <211> 10
   <212> PRT
   <213> künstliche Sequenz
<220>
   <223> mutated VP3 of adeno-associated virus
<400> 14
<210> 15
   <211> 10
   <212> PRT
   <213> künstliche Sequenz
<220>
   <223> mutated VP3 of adeno-associated virus
<400> 15
<210> 16
   <211> 10
   <212> PRT
   <213> künstliche Sequenz
<220>
   <223> mutated VP3 of adeno-associated virus
<400> 16
<210> 17
   <211> 4
   <212> PRT
   <213> Mus musculus
<400> 17
   Arg Gly Asp Ser
   4
<210> 18
   <211> 4
   <212> PRT
   <213> künstliche Sequenz
<220>
   <223> mutated laminin peptide
<400> 18
   Arg Gly Glu Ser
   4

## Patentansprüche

1. Verwendung eines Strukturproteins von Adeno-assoziiertem Virus (AAV) für die Aufreinigung von AAV und/oder AAV-Partikeln, **dadurch gekennzeichnet, daß** das Strukturprotein mindestens eine Mutation in Form einer Insertion enthält, die eine Änderung der chromatographischen Eigenschaften des Virus bewirkt und die nach mindestens einer Aminosäure in der Sequenz ausgewählt aus YKQIS SQSGA, YLTLN NGSQA, YYLSR TNTPS, EEKFF PQSGV, NPVAT EQYGS, LQRGN RQAAT, NVDFT VDTNG lokalisiert ist.

2. Verwendung eines Strukturproteins nach Anspruch 1, **dadurch gekennzeichnet, daß** die Änderung der chromatographischen Eigenschaften eine Verbesserung der Aufreinigung ermöglicht, insbesondere eine Anreicherung des Virus, vorzugsweise der Virus-Partikel, zu höheren Titern, eine Aufreinigung zu größerer Reinheit und/oder eine effizientere Aufreinigung.

3. Verwendung eines Strukturproteins nach einem der Ansprüche 1 oder 2, **dadurch gekennzeichnet, daß** die Mutation keine wesentliche Verringerung der Infektiosität des Virus bewirkt.

4. Verwendung eines Strukturproteins nach einem der Ansprüche 1 bis 3, **dadurch gekennzeichnet, daß** das mutierte Strukturprotein zur Partikelbildung befähigt ist.

5. Verwendung eines Strukturproteins nach einem der Ansprüche 1 bis 4, **dadurch gekennzeichnet, daß** das mutierte Strukturprotein die Hitzestabilität erhöht.

6. Verwendung eines Strukturproteins nach einem der Ansprüche 1 bis 5, **dadurch gekennzeichnet, daß** es ausgewählt ist aus mutiertem VP1, mutiertem VP2 und/oder mutiertem VP3.

7. Verwendung eines Strukturproteins nach einem der Ansprüche 1 bis 6, **dadurch gekennzeichnet, daß** es abgeleitet ist von AAV1, AAV2, AAV3, AAV4, AAV 5 und/oder AAV6 sowie anderen von diesen, insbesondere von AAV2, abgeleiteten AAV-Serotypen.

8. Verwendung eines Strukturproteins nach einem der Ansprüche 1 bis 7, **dadurch gekennzeichnet, daß** Aminosäuren einer funktionellen Sequenz insertiert werden, die vorzugsweise für die Affinitätschromatographie geeignet sind.

9. Verwendung eines Strukturproteins nach Anspruch 8, **dadurch gekennzeichnet, daß** die insertierte Aminosäuresequenz ausgewählt ist aus einem Liganden eines Rezeptors oder dem Rezeptor eines Liganden, einem Antikörper oder Teil eines Anitikörpers, insbesondere einem Antikörper-Epitop, einem Antigen oder Antigen-Epitop, einem Hormon, einem Hormorezeptor, einem Enzym, einem Enzymsubstrat, einem Lektin, zucker-tragenden Aminosäuren, insbesondere aus einem Histidin-reichen Peptid (His-Tag), einem mehrfach geladenen Peptid, der Glutathion-S-Transferase (GST-Tag), einem F_{c}-Teil eines Antikörpers, einer Immunglobulin-bindenden Domäne, beispielsweise Protein A oder Protein G oder ein Teil davon, einem Lecitin, einer Nukleinsäure-Bindestelle, einer Heparin-Bindestelle, einem spezifischen Liganden, einem spezifischen Rezeptor, einem Integrin, einem Cytokin oder einer Rezeptor-Bindungsdomäne von einem Cytokin, Integrin oder Wachstumsfaktor, an einem Zelloberflächenrezeptor bindenden einzelkettigen Antikörper, einem Antikörper gegen Zelloberflächenstrukturen, einem Epitop und/oder einer antikörperbindenden Struktur.

10. Verwendung eines Strukturproteins nach einem der Ansprüche 8 oder 9, **dadurch gekennzeichnet, daß** ein Peptid insertiert wird, das die Sequenz QAGTFALRGDNPQG hat.

11. Verwendung eines Strukturproteins nach einem der Ansprüche 1 bis 10, **dadurch gekennzeichnet, daß** das Strukturprotein mindestens eine weitere Mutation enthält.

12. Verwendung eines Strukturproteins nach Anspruch 11, **dadurch gekennzeichnet, daß** die weitere(n) Mutation(en) eine Änderung der Infektiosität des Virus bewirkt.

13. Verwendung eines Strukturproteins nach einem der Ansprüche 11 oder 12, **dadurch gekennzeichnet, daß** die weitere(n) Mutation(en) eine Verringerung der Antigenität des Virus bewirkt.

14. Verwendung eines Strukturproteins nach einem der Ansprüche 11 bis 13, **dadurch gekennzeichnet, daß** die weitere(n) Mutation(en) eine oder mehrere Deletion(en), eine oder mehrere Insertion(en) oder eine Kombination der genannten Modifikationen ist/sind.

15. Verwendung eines Strukturproteins nach einem der Ansprüche 11 bis 14, **dadurch gekennzeichnet, daß** die Insertion ein Zellmembranrezeptor-Ligand, ein Rep-Protein oder -Peptid, ein immunsuppressives Protein oder Peptid und/oder ein Protein oder Peptid mit einem Signal zur Doppelstrangsynthese des Fremdgens ist.

16. Verwendung eines Strukturproteins nach einem der Ansprüche 11 bis 15, **dadurch gekennzeichnet, daß** die Insertion ausgewählt ist aus einem Integrin, einem Cytokin oder einer Rezeptor-Bindungsdomäne von einem Cytokin, Integrin oder Wachstumsfaktor, an einem Zelloberflächenrezeptor bindenden einzelkettigen Antikörper, einem Antikörper gegen Zelloberflächenstrukturen, einer antikörperbindenden Struktur oder einem Epitop.

17. Verwendung eines Strukturproteins gemäß einem der Ansprüche 1 bis 16 in Form eines AAV-Partikels, insbesondere in Form eines AAV-Kapsids.

## Claims

1. Use of a structural protein of adeno-associated virus (AAV) for the purification of AAV and/or AAV particles, **characterised in that** the structural protein contains at least one mutation in the form of an insertion which brings about a change in the chromatographic properties of the virus and which is located after at least one amino acid in the sequence selected from YKQIS SQSGA, YLTLN NGSQA, YYLSR TNTPS, EEKFF PQSGV, NPVAT EQYGS, LQRGN RQAAT, NVDFT VDTNG.

2. Use of a structural protein according to claim 1, **characterised in that** the change in the chromatographic properties facilitates an improvement in purification, especially enrichment of the virus, especially of the virus particles, to higher titres, purification to greater purity and/or more efficient purification.

3. Use of a structural protein according to either one of claims 1 and 2, **characterised in that** the mutation does not bring about any appreciable reduction in the infectivity of the virus.

4. Use of a structural protein according to any one of claims 1 to 3, **characterised in that** the mutated structural protein is capable of particle formation.

5. Use of a structural protein according to any one of claims 1 to 4, **characterised in that** the mutated structural protein increases thermal stability.

6. Use of a structural protein according to any one of claims 1 to 5, **characterised in that** it is selected from mutated VP1, mutated VP2 and/or mutated VP3.

7. Use of a structural protein according to any one of claims 1 to 6, **characterised in that** it is derived from AAV1, AAV2, AAV3, AAV4, AAV5 and/or AAV6 as well as from other AAV serotypes derived therefrom, especially from AAV2.

8. Use of a structural protein according to any one of claims 1 to 7, **characterised in that** there are inserted amino acids of a functional sequence that are especially suitable for affinity chromatography.

9. Use of a structural protein according to claim 8, **characterised in that** the inserted amino acid sequence is selected from a ligand of a receptor or the receptor of a ligand, an antibody or part of an antibody, especially an antibody epitope, an antigen or antigen epitope, a hormone, a hormone receptor, an enzyme, an enzyme substrate, a lectin, sugar-bearing amino acids, especially from a histidine-rich peptide (His-Tag), a multiply charged peptide, glutathione-S-transferase (GST-Tag), an F_{c} portion of an antibody, an immunoglobulin-binding domain, for example protein A or protein G or a portion thereof, a lecithin, a nucleic-acid-binding site, a heparin-binding site, a specific ligand, a specific receptor, an integrin, a cytokine or a receptor-binding domain of a cytokine, integrin or growth factor, single-chain antibodies binding to a cell surface receptor, an antibody to cell surface structures, an epitope and/or an antibody-binding structure.

10. Use of a structural protein according to either one of claims 8 and 9, **characterised in that** there is inserted a peptide which has the sequence QAGTFALRGDNPQG.

11. Use of a structural protein according to any one of claims 1 to 10, **characterised in that** the structural protein contains at least one further mutation.

12. Use of a structural protein according to claim 11, **characterised in that** the further mutation(s) bring about a change in the infectivity of the virus.

13. Use of a structural protein according to either one of claims 11 and 12, **characterised in that** the further mutation(s) bring about a reduction in the antigenicity of the virus.

14. Use of a structural protein according to any one of claims 11 to 13, **characterised in that** the further mutation(s) is/are one or more deletion(s), one or more insertion(s) or a combination of the said modifications.

15. Use of a structural protein according to any one of claims 11 to 14, **characterised in that** the insertion is a cell membrane receptor ligand, a Rep protein or Rep peptide, an immunosuppressive protein or peptide and/or a protein or peptide having a signal for double-strand synthesis of the foreign gene.

16. Use of a structural protein according to any one of claims 11 to 15, **characterised in that** the insertion is selected from an integrin, a cytokine or a receptor-binding domain of a cytokine, integrin or growth factor, single-chain antibodies binding to a cell surface receptor, an antibody to cell surface structures, an antibody-binding structure or an epitope.

17. Use of a structural protein according to any one of claims 1 to 16 in the form of an AAV particle, especially in the form of an AAV capsid.

## Revendications

1. Utilisation d'une protéine structurale de virus adéno-associé (AAV) pour la purification d'AAV et/ou de particules d'AAV, **caractérisée en ce que** la protéine structurale contient au moins une mutation sous la forme d'une insertion, qui provoque une modification des propriétés chromatographiques du virus et qui est située à la suite d'au moins un acide aminé dans la séquence consistant en YKQIS SQSGA, YLTLN NGSQA, YYLSR TNTPS, EEKFF PQSGV, NPVAT EQYGS, LQRGN RQAAT ou NVDFT VDTNG.

2. Utilisation d'une protéine structurale selon la revendication 1, **caractérisée en ce que** la modification des propriétés chromatographiques permet une amélioration de la purification, en particulier un enrichissement du virus, de préférence des particules de virus, à un niveau plus élevé, une purification à une pureté plus grande et/ou une purification plus efficace.

3. Utilisation d'une protéine structurale selon l'une quelconque des revendications 1 ou 2, **caractérisée en ce que** la mutation ne provoque pas de diminution considérable de l'infectivité du virus.

4. Utilisation d'une protéine structurale selon l'une quelconque des revendications 1 à 3, **caractérisée en ce que** la protéine structurale mutée est capable de former des particules.

5. Utilisation d'une protéine structurale selon l'une quelconque des revendications 1 à 4, **caractérisée en ce que** la protéine structurale mutée augmente la stabilité thermique.

6. Utilisation d'une protéine structurale selon l'une quelconque des revendications 1 à 5, **caractérisée en ce que** ladite protéine est choisie dans le groupe comprenant la VP1 mutée, la VP2 mutée et/ou la VP3 mutée.

7. Utilisation d'une protéine structurale selon l'une quelconque des revendications 1 à 6, **caractérisée en ce qu'**elle est dérivée d'AAV1, AAV2, AAV3, AAV4, AAV5 et/ou AAV6 ainsi que d'autres de ces sérotypes d'AAV dérivés de ceux-ci, en particulier d'AAV2.

8. Utilisation d'une protéine structurale selon l'une quelconque des revendications 1 à 7, **caractérisée en ce que** des acides aminés d'une séquence fonctionnelle y sont insérés, lesquels acides aminés de préférence conviennent pour la chromatographie d'affinité.

9. Utilisation d'une protéine structurale selon la revendication 8, **caractérisée en ce que** la séquence d'acides aminés insérée est choisie parmi un ligand d'un récepteur ou le récepteur d'un ligand, un anticorps ou un fragment d'anticorps, en particulier un épitope pour un anticorps, un antigène ou un épitope d'antigène, une hormone, un récepteur hormonal, une enzyme, un substrat d'enzyme, une lectine, des acides aminés portant des glucides, en particulier un peptide riche en histidine (His-Tag), un peptide à plusieurs charges, la glutathion-S-transférase (GST-Tag), un fragment F_{c} d'un anticorps, un domaine de liaison aux immunoglobulines, par exemple la protéine A ou la protéine G ou un fragment de celle-ci, une lécithine, un site de liaison aux acides nucléiques, un site de liaison à l'héparine, un ligand spécifique, un récepteur spécifique, une intégrine, une cytokine ou un domaine de liaison aux récepteurs d'une cytokine, d'une intégrine ou d'un facteur de croissance, un anticorps à une seule chaîne se liant à un récepteur de surface cellulaire, un anticorps dirigé contre des structures de surfaces cellulaires, un épitope et/ou une structure de liaison aux anticorps.

10. Utilisation d'une protéine structurale selon l'une quelconque des revendications 8 ou 9, **caractérisée en ce qu'**y est inséré un peptide de séquence QAGTFALRGDNPQG.

11. Utilisation d'une protéine structurale selon l'une quelconque des revendications 1 à 10, **caractérisée en ce que** la protéine structurale contient au moins une autre mutation.

12. Utilisation d'une protéine structurale selon la revendication 11, **caractérisée en ce que** la ou les autres mutations provoquent une modification de l'infectivité du virus.

13. Utilisation d'une protéine structurale selon l'une quelconque des revendications 11 ou 12, **caractérisée en ce que** la ou les autres mutations provoquent une diminution considérable de l'antigénicité du virus.

14. Utilisation d'une protéine structurale selon l'une quelconque des revendications 11 à 13, **caractérisée en ce que** la ou les autres mutations sont une ou plusieurs délétions, une ou plusieurs insertions ou une combinaison des modifications citées.

15. Utilisation d'une protéine structurale selon l'une quelconque des revendications 11 à 14, **caractérisée en ce que** l'insertion est un ligand de récepteur membranaire, une protéine ou un peptide Rep, une protéine ou un peptide immunosuppresseur et/ou une protéine ou un peptide avec un signal pour la synthèse en double brin du gène étranger.

16. Utilisation d'une protéine structurale selon l'une quelconque des revendications 11 à 15, **caractérisée en ce que** l'insertion est choisie parmi une intégrine, une cytokine ou un domaine de liaison aux récepteurs d'une cytokine, d'une intégrine ou d'un facteur de croissance, un anticorps à une seule chaîne se liant à un récepteur de surface cellulaire, un anticorps dirigé contre des structures de surfaces cellulaires, une structure de liaison aux anticorps ou un épitope.

17. Utilisation d'une protéine structurale selon l'une quelconque des revendications 1 à 16 sous la forme d'une particule d'AAV, en particulier sous la forme d'une capside d'AAV.
